# EUROPEAN PATENT SPECIFICATION

(11) **EP 2 904 114 B1**
(45) Date of publication and mention of the grant of the patent: **30.11.2016**
(21) Application number: 13798365.6
(22) Date of filing: 04.10.2013
(51) Int. Cl.: C12Q 1/68

(54) **METHOD OF DETECTING A SINGLE NUCLEOTIDE TARGET**
VERFAHREN ZUR DETEKTION EINES EINZELNEN NUKLEOTIDS
PROCÉDÉ POUR DÉTECTER UN NUCLÉOTIDE SIMPLE

(30) Priority: 04.10.2012 GB 201217770
(43) Date of publication of application: 12.08.2015
(73) Proprietor: Base4 Innovation Ltd, Cambridge, Cambridgeshire CB3 0FA (GB)
(72) Inventor: FRAYLING, Cameron Alexander, Cambridge Cambridgeshire CB3 0FA (GB); BALMFORTH, Barnaby, Cambridge Cambridgeshire CB3 0FA (GB); SOARES, Bruno Flavio Nogueira de Sousa, Cambridge Cambridgeshire CB3 0FA (GB); ISAAC, Thomas Henry, Cambridge Cambridgeshire CB3 0FA (GB); BREINER, Boris, Cambridge Cambridgeshire CB3 0FA (GB); NATALE, Alessandra, Cambridge Cambridgeshire CB3 0FA (GB); AMASIO, Michele, Cambridge Cambridgeshire CB3 0FA (GB)
(74) Representative: Lau, Sarah Jane
(86) International application number: PCT/GB2013/052594
(87) International publication number: WO 2014/053853

(56) References cited:
- EP-A2- 0 745 690
- WO-A2-2006/115570
- WO-A2-2012/071344
- US-A- 5 268 266
- US-A1- 2003 058 799
- MEDHURST A D ET AL: "The use of TaqMan RT-PCR assays for semiquantitative analysis of gene expression in CNS tissues and disease models", JOURNAL OF NEUROSCIENCE METHODS, ELSEVIER SCIENCE PUBLISHER B.V., AMSTERDAM, NL, vol. 98, no. 1, 15 May 2000 (2000-05-15), pages 9-20, XP002336884, ISSN: 0165-0270, DOI: 10.1016/S0165-0270(00)00178-3
- JOHN EID ET AL: "Real-time DNA sequencing from single polymerase molecules", SCIENCE, AMERICAN ASSOCIATION FOR THE ADVANCEMENT OF SCIENCE, WASHINGTON, DC; US, vol. 323, no. 5910, 2 January 2009 (2009-01-02), pages 133-138, XP008143766, ISSN: 0036-8075, DOI: 10.1126/SCIENCE.1162986
- MITRA R D ET AL: "Fluorescent in situ sequencing on polymerase colonies", ANALYTICAL BIOCHEMISTRY, ACADEMIC PRESS INC, NEW YORK, vol. 320, no. 1, 1 September 2003 (2003-09-01), pages 55-65, XP004441395, ISSN: 0003-2697, DOI: 10.1016/S0003-2697(03)00291-4
- C. SHI ET AL: "Anti-gene padlocks eliminate Escherichia coli based on their genotype", JOURNAL OF ANTIMICROBIAL CHEMOTHERAPY, vol. 61, no. 2, 19 December 2007 (2007-12-19), pages 262-272, XP055095879, ISSN: 0305-7453, DOI: 10.1093/jac/dkm482

## Description

The present invention relates to methods for detecting the presence of complimentary single nucleotides as defined in appended claims 1-10.

Biological probes, which typically comprise single-stranded oligonucleotides of known sequence order less than 1000 nucleotides long, are widely used in analytical molecular biology. Such probes typically work by attaching themselves to the target (for example one derived from the DNA of a naturally-occurring pathogen) when complete or sufficiently complete sequence complimentarity exists between the nucleotide bases of the probe and the target. Typically, the nucleotides of such probes are labelled with detectable elements such as radioactive or fluorescent markers so that when the probe is used to treat an analyte solution or substrate in or on which the target is thought to have been captured, the presence or absence of the target is revealed by searching for and detecting the detection element's characteristic detection property.

One class of such probes is represented by materials known in the art as 'molecular beacons'; as for example described in WO02/06531 or US 8211644. These probes are comprised of single-stranded oligonucleotides which have been in effect folded back onto themselves to create a residual single-stranded loop which acts as the probe's sensor and a short stem where the nucleotides adjacent the two ends are bound to each other through complimentary nucleotide base pairing; thereby creating a double-stranded region. This arrangement, which can be likened to a hairpin in which the single-stranded loop is attached to complimentary strands of the same end of a notional double-stranded oligonucleotide (i.e. the stem), is highly strained. To the free 3' and 5' ends of the oligonucleotide (now adjacent to one another and at the remote end of the stem) are attached respectively a fluorophore and a quencher. Their proximity to each other ensures that no significant fluorescence occurs. In use, the target binds to the single-stranded loop causing additional strain which then causes the stem to unzip thereby distancing the fluorophore and quencher and allowing the former to fluoresce. One disadvantage of these probes is that the loop needs to be relatively long, e.g. 20 to 30 nucleotides, making them unsuitable for detecting smaller targets and especially those comprising single nucleotides.

US 2006/063193 describes a detection method comprising contacting an unknown single-stranded analyte with an array of different probe types each having a different sequence; hybridising the analyte to its complimentary probe and determining the sequence by performing mass spectroscopy on the hybridised probe. This method however, which is different from that of the present invention, is not especially suitable for the identification of single nucleotides.

EP 1662006 teaches a DNA probe derived from two complimentary oligonucleotide strands of differing lengths the longer of which is designed to be the sequence compliment of a single-stranded target. In its unused state, the probe therefore comprises a double-stranded region and a single-stranded region which can recognise and become partially attached to the target by hybridisation. Thereafter, the shorter of the two strands in the probe and the remainder of the sequence of the target can be exchanged enabling the analyte to become fully hybridised to the probe. In one embodiment, corresponding nucleotides on the two strands of the probe are functionalised with pairs of fluorophores and quenchers rendering the unused probe non-fluorescing. When however the shorter strand is exchanged away by the remainder of the target the fluorophores are freed up to fluoresce.

WO 2006/071776 describes a ligation-based RNA amplification method involving the use of a nucleic acid comprising a double-stranded region and a single-stranded 3' terminal region. In this method the 5' end of the RNA is attached to the single-stranded region and the 3' end to the strand of the double-stranded region which is 5'. Thereafter the RNA can be amplified using known techniques. However the nucleic acid does not appear to be labelled with detectable elements.

WO 2009/120372 teaches a method in which a double-stranded oligonucleotide of unknown sequence is first converted to a template nucleic acid by first attaching first and second hairpin single-stranded regions to the ends thereof. The template so produced can then be used to simultaneously sequence the double-stranded oligonucleotide in both the sense and antisense directions using a conventional polymerase mediated sequencing method involving priming the hairpins; separating the constituent strands of the double-strand oligonucleotide: and extending the primers along the separated strands. However, none of the nucleotides within in the template appear to be labelled with detectable elements.

WO 2006/115570 discloses a detector probe comprising a double-stranded stem-loop structure, a target-complementary single-stranded region, a further double-stranded region and two reporter molecules suitable for the detection of small nucleic acid molecules.

US 2003/058799 discloses a method of detecting a single nucleotide that has been removed from a larger target nucleic acid molecule by exonuclease activity by Raman spectroscopy or by fluorescence resonance energy transfer spectroscopy.

We have now developed alternative biological probes in which the detectable elements are essentially undetectable unless specifically activated by a sequence of biochemical/enzymatic reactions which liberate one or a cascade of the detectable elements from the probe in a more easily detectable state. Such biological probes are useful in situations where the concentration of the target is very small and especially so where the target comprises a stream of single nucleotides whose associated nucleotide base ordering corresponds to that of an unknown biomolecule whose sequence needs to be determined. This opens up the possibility of using the probes of the present disclosure in high throughput DNA sequencing devices.

The present disclosure provides a biological probe characterised in that it comprises a single-stranded nucleotide region the ends of which are each attached to two different double-stranded oligonucleotide regions wherein at least one of the oligonucleotide regions comprises detectable elements having a characteristic detection property and wherein the detectable elements are so arranged on the oligonucleotide region that the detectable property is less detectable than when the same number of detectable elements are bound to a corresponding number of single nucleotides.

In one preferred aspect the detectable elements comprise fluorophores and the probe itself is essentially non-fluorescing at those wavelengths where the fluorophores are designed to be detected. Thus, although a fluorophore may exhibit general, low-level background fluorescence across a wide part of the electromagnetic spectrum there will typically be one or a small number of specific wavelengths or wavelength envelopes where the intensity of the fluorescence is at a maximum. It is at one or more of these maxima where the fluorophore is characteristically detected that essentially no fluorescence should occur. In the context of the present disclosure by the term 'essentially non-fluorescing' or equivalent wording is meant that the intensity of fluorescence of the total number of fluorophores attached to the probe at the relevant characteristic wavelength(s) or wavelength envelope is less than 25%; preferably less than 10%; more preferably less than 1% and most preferably less than 0.1% of the corresponding intensity of fluorescence of an equivalent number of free fluorophores.

In principle, any method can be used to ensure that in the probe's unused state the fluorophores fluoresce less than when each are bound to their own single nucleotide. One approach is to additionally attach quenchers in close proximity thereto. Another is based on the observation that when multiple fluorophores are attached to the same probe in close proximity to each other they tend to quench each other sufficiently well that the criterion described in the previous paragraph can be achieved without the need for quenchers. In this context of this patent, what constitutes 'close proximity' between fluorophores or between fluorophores and quenchers will depend on the particular fluorophores and quenchers used and possibly the structural characteristics of the oligonucleotide region(s). Consequently, it is intended that this term be construed with reference to the required outcome rather than any particular structural arrangement on the probe. However, and for the purposes of providing exemplification, it is pointed out that when adjacent fluorophores or adjacent fluorophores and quenchers are separated by a distance corresponding to their characteristic Förster distance (typically less than 5nm) sufficient quenching will be achieved.

Preferably at least one of the oligonucleotide regions which comprise the probe is labelled with up to 20, preferably up to 10 and most preferably up to 5 fluorophores. To obtain maximum advantage, it is preferred that at least one of the oligonucleotide regions is labelled with at least 2 preferably at least 3 fluorophores. Consequently, ranges constructed from any permutation of these maxima and minima are specifically envisaged herein. If quenchers are employed, it is likewise preferred that the probe is labelled with up to 20, preferably up to 10 and most preferably up to 5 of the same. Whilst it is envisaged that more than one type of fluorophore can be attached to the probe, for example to give it a characteristic fingerprint, it is preferred that all the fluorophores attached to a given probe are of the same type. Preferably the fluorophores and quenchers are on different strands of the oligonucleotide region or opposite each other where they are created by folding a single-stranded oligonucleotide precursor.

As regards the fluorophores themselves they can in principle chosen from any of those conventionally used in the art including but not limited to xanthene moieties e.g. fluorescein, rhodamine and their derivatives such as fluorescein isothiocyanate, rhodamine B and the like; coumarin moieties (e.g. hydroxy-, methyl- and aminocoumarin) and cyanine moieties such as Cy2, Cy3, Cy5 and Cy7. Specific examples include fluorophores derived from the following commonly used dyes: Alexa dyes, cyanine dyes, Atto Tec dyes, and rhodamine dyes. Examples also include: Atto 633 (ATTO-TEC GmbH), Texas Red, Atto 740 (ATTO-TEC GmbH), Rose Bengal, Alexa Fluor™ 750 C₅-maleimide (Invitrogen), Alexa Fluor™ 532 C₂-maleimide (Invitrogen) and Rhodamine Red C₂-maleimide and Rhodamine Green as well as phosphoramadite dyes such as Quasar 570. Alternatively a quantum dot or a near infra-red dye such as those supplied by LI-COR Biosciences can be employed. The fluorophore is typically attached to the oligonucleotide via a nucleotide base using chemical methods known in the art.

Suitable quenchers are those which work by a Förster resonance energy transfer (FRET) mechanism. Non-limiting examples of commercially available quenchers which can be used in association with the above mentioned-fluorophores include but are not limited to DDQ-1, Dabcyl, Eclipse, Iowa Black FQ and RQ, IR Dye -QC1, BHQ-1, -2 and -3 and QSY-7 and -21.

Turning to the single-stranded nucleotide region of the probe this can be up to 1000 nucleotides preferably up to 300 nucleotides long and either generated *ab initio* by chemical synthesis or derived from a naturally-occurring source such as bacterial DNA. In one aspect of the disclosure the nucleotide region is suitably up to 100 nucleotides in length preferably up to 50 nucleotides and most preferably up to 30 nucleotides. In the methods of the invention the single-stranded region is comprised of one nucleotide only making the probe extremely selective for the detection of the free nucleotide having a complimentary nucleotide base. In the case of targets derived from naturally-occurring DNA or RNA this opens up the possibility of employing a multicomponent biological probe mixture comprising up to four different biological probes each selective for a different nucleotide base characteristic of the target (i.e. for DNA one of guanine, cytosine, adenine and thymine or for RNA one of guanine, cytosine, adenine and uracil) and each employing a different detectable element; in particular different fluorophores fluorescing at different characteristic wavelengths or wavelength envelopes.

Turning to the double-stranded oligonucleotide region(s), it is preferred that they are derived or derivable from two oligonucleotide precursors, each preferably closed looped at the end remote from the single-stranded nucleotide region, or from a common single-stranded oligonucleotide precursor by folding the latter's two ends back on themselves to create two closed loop oligonucleotide regions with an intermediate gap constituting the single-stranded nucleotide region. In all cases the effect is the same; adjacent to the ends of the single-stranded nucleotide region will be 3' and 5' free ends on the other strand of the oligonucleotide region to which the corresponding 5' and 3' ends of the target can be attached. Thus use of the probe involves a process of attaching the single-stranded nucleotide region to a target having a complimentary sequence of nucleotide bases by joining up to said 3' and 5' ends to generate a used probe which is double-stranded along it whole length.

Where the biological probe is comprised of two discrete double-stranded oligonucleotides it is preferred that each end remote from the nucleotide region is a closed loop. Suitably, the oligonucleotide region(s) are up to 50 nucleotide pairs long, preferably up to 45 nucleotide pairs, more preferably in the range 5 to 40 nucleotide pairs and most preferably in the range 10 to 30 nucleotides. Longer oligonucleotide regions may be used but the potential risk that access to the nucleotide region may become restricted through becoming entangled with them makes this embodiment less attractive.

It is preferred that the detectable elements bound to the oligonucleotide regions are located remote from the nucleotide region. Where two discrete oligonucleotides regions are employed it is preferred that the detectable elements are located or clustered at or towards one or both of the ends thereof which are remote from the nucleotide region. In one preferred embodiment at least one of the oligonucleotide regions comprises a restriction enzyme recognition site preferably adjacent the region where the detectable elements are located or clustered. Such a restriction enzyme recognition site will typically comprise a specific sequence of from 2 to 8 nucleotide pairs. In another preferred embodiment the restriction enzyme recognition site is created by binding of the target to the nucleotide region.

The biological probes of the present disclosure can in principle be manufactured by any of the nucleotide assembly methodologies known in the art including the H-phosphonate method, the phosophodiester synthesis, the phosphotriester synthesis and the phosphite triester synthesis. Preferred, are methods employing nucleotide phosphoramadite building blocks on account of their reactivity. In these methods synthesis occurs by sequential addition of the chosen nucleotide phosphoramadite to the growing nucleotide chain at the 5' position in a cyclic four-step process involving de-blocking, coupling, capping and oxidation. The cyclic nature of this process makes it especially amenable to automation and machines to do this are readily available on the market. Where quenchers and/or fluorophores are to be introduced the appropriately labelled nucleotide phosphoramadite is employed at the required point. In a most preferred embodiment, the phosphoramadite method is used to make a single-stranded oligonucleotide precursor which is folded by a cycle of rapid heating and slow cooling into a probe having the desired characteristics.

The probes are typically utilised in solution but can if so desired be advantageously be immobilised on a substrate such as a polymer, membrane, chip array and the like or in a nanopore or a nanochannel.

In a second aspect of the disclosure there is provided a method for using the biological probe to detect a target characterised by comprising the step of (a) attaching the target to the single-stranded nucleotide region of a biological probe of the type descried above to create a used probe which is wholly double-stranded. Typically this step (a) is caused to take place by means of a polymerase which binds the 5' end of the target to a 3' end of the oligonucleotide and a ligase to join the remaining free ends of the target and the oligonucleotide or other oligonucleotide together. A wide range of polymerases and ligases can be used including but are not limited to those derived from readily-available bacterial sources such as bacteriophage T4, *Escherichia Coli* and *Thermus Aquaticus* (Taq). Preferably step (a) is carried out in an aqueous medium in the presence of excess probe with suitably the molar ratio of target to probe being in the range 1:1 to 1:2000, preferably 1:1 to 1: 200, more preferably 1:2 to 1:50 and with 1:5 to 1:20 being most preferred. Suitably, the target is a single nucleotide or a single-stranded oligonucleotide having a nucleotide base or nucleotide base sequence complimentary to that of the nucleotide region on the biological probe. In the methods of the invention, the target is a single nucleotide characteristic of naturally occurring DNA or RNA. A stoichiometric excess of each of the two enzymes over the target is suitably employed when the reaction medium is dilute.

Preferably, the method of the present invention further comprises the step of (b) treating the used probe obtained in step (a) with a restriction enzyme (restriction endonuclease) and an exonuclease to liberate the detectable element(s) therefrom and in a form in which can be detected. Thereafter, in a step (c) the detectable element(s) so liberated are detected by observing the detectable property associated therewith. Thus, when the detectable elements in the probe are relatively non-fluorescing fluorophores, step (b) liberates them in a form which enables them to fluoresce optimally. This fluorescence can then be detected and measured using conventional techniques to provide an output data set or data stream which can be used for analytical purposes. In step (b) this liberation of the fluorophores comes about by first the restriction enzyme making a double-stranded cut in the used probe at the restriction enzyme recognition site mentioned above. The short fragments so created are then degraded further by the exonuclease into single nucleotides at least some of which will be labelled with fluorophores. When the probe comprises multiple fluorophores this leads to a cascade of liberated fluorophores which, by virtue of them now being separated from each other or their associated quenchers, are now free to fluoresce in the normal way. Preferably, this fluorescence is detected in step (c) by a photodetector or an equivalent device tuned to the characteristic fluorescence wavelength or wavelength envelope of the fluorophore. This in turn causes the photodetector to generate an electrical signal which can be processed and analysed in the normal way. Typically step (b) is also carried out in an aqueous medium with an excess of enzymes. In order to avoid degrading unused biological probe it is preferred that the restriction enzyme recognition site is that formed by adding the target to the single-stranded nucleotide region or alternatively that the restriction enzyme is chosen so that it will not react with double-stranded oligonucleotides which contain nicks therein. The restriction enzyme will thus be chosen with the characteristics of the restriction enzyme site in mind and will in particular be one which shows high fidelity for the site if the probes are to perform optimally. Suitable exonucleases include Dnase I (RNase-free), Exonuclease I or III (ex E.Coli), Exonuclease T, Exonuclease V (RecBCD), Lambda Exonuclease, Micrococcal Nuclease, Mung Bean Nuclease, Nuclease BAL-31 RecJ_{f} T5 Exonuclease and T7 Exonuclease.

One preferred use of the method is where the target is a single nucleotide and where it is contacted with a mixture of four different biological probes each having a single-stranded nucleotide region comprising a different single nucleotide selected from those whose associated nucleotide base comprises (1) guanine, cytosine, adenine and thymine or (2) guanine, cytosine, adenine and uracil. However other nucleotides corresponding to other nucleotide bases (e.g. those constitutive of other synthetic polynucleotides) can be employed if so desired. In all cases it is preferred that each probe has a different associated detectable element preferably a different fluorophore. In a most preferred embodiment, the target comprises a stream of single nucleotides the ordering of which corresponds to the sequence of nucleotides in a DNA or RNA sample whose sequence is unknown or only partially known. By such means the method can provide the basis for the design and operation of a DNA or RNA sequencing device.

The present invention will now be illustrated by the following Examples and Figures.

### Example 1

A 103 nucleotide single-stranded oligonucleotide precursor (ex. ATDBio) having the nucleotide base sequence: 
(5')GGCACGATGGXXAXXGCCCGCACTTCAGCGGGCAAYAACCATCGTGCCTGCAGGCTCGACCTTTATTCG
CGGCACTTCAGCCGCGAATAAAGGTCGAGCCTGC(3')
 wherein X are T bases labelled with Quasar 570 (fluorophore) and wherein Y are T bases labelled with BHQ-2 quencher, is folded about the 49^{th} nucleotide base by heating an aqueous solution of it to 95°C and then cooled slowly back to room temperature at a rate of 10 minutes per °C. At the end of this time, a closed-loop ended probe according to the present invention is formed in which the 49^{th} nucleotide base (here T) comprises the single-stranded nucleotide region and two double-stranded oligonucleotides, respectively 24 and 27 nucleotide base pairs long, flank it.

### Examples 2 to 4

The method of Example 1 is repeated three further times except that the 103 nucleotide precursor is modified so that nucleotide base in the 49^{th} position is G (Example 2), C (Example 3) and A (Example 4) to create three further probes selective for different nucleotide bases. The X bases used in each of these examples are T bases labelled respectively with: Fluorescein (517nm), Texas Red (612nm) and cyanine-5 (667nm).

### Example 5

A probe mixture is created by mixing equimolar amounts of the four probes of Examples 1 to 4 in aqueous solution at room temperature.

### Example 6

The mixture of Example 5 is interrogated with 547 nanometre laser light and the degree of fluorescence measured at 570nm using a photodetector. Thereafter an aqueous solution comprising single nucleotides having associated A bases is added (molar ratio of single nucleotide to probe 1: 50) along with catalytic amounts of Klenow fragment polymerase (produced from DNA polymerase I (ex. E. Coli) and subtilisin) and E.coli DNA ligase. After one hour, the fluorescence is again measured before an aqueous solution of restriction enzyme Sbf1 and Lambda Exonuclease is added in catalytic amounts. After a further hour has elapsed the degree of fluorescence is again measured. The fluorescence measured at 570nm in the first two instances is found to be less that 99% of that measured in the last.

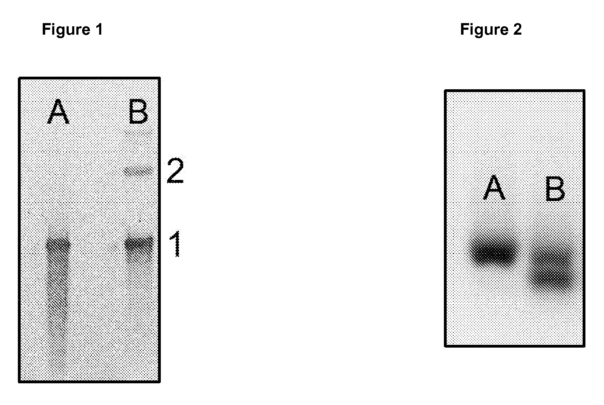

Figure 1 uses gel chromatographic data to illustrate the working of the nucleotide-capture step. Lane A shows a result obtained from a sample of an unused probe of the type described above. Lane B shows a result from a similar sample after the relevant nucleic acid (in dNTP form), polymerase and ligase have been added. The presence of a second band, indicative of the presence of the completed probe, can be seen.
Figure 2 uses gel chromatographic data to illustrate the working of a restriction endonuclease in cleaving the completed probe. Lane A shows the completed probe and Lane B shows the situation after the probe has been in contact with the restriction enzyme for a period of time. The presence of multiple bands in Lane B is indicative of the fact that cleavage into two fragments has taking place.

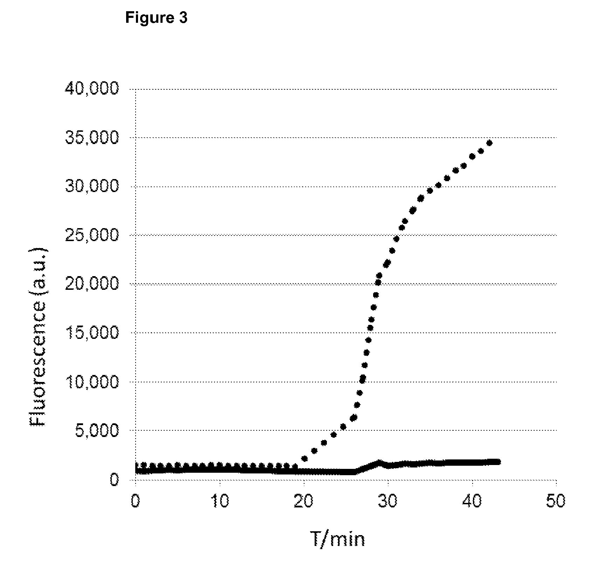

Finally, Figure 3 graphically shows the development of fluorescence over time after a cleaved 'dark' completed probe of the type described above is subject to progressive exonucleolytic degradation to release its constituent fluorophores in an active state.

## Claims

1. A method of detecting a single nucleotide target **characterised in that** it comprises the step of attaching said target to a probe using a polymerase and a ligase to create a used probe which is wholly double-stranded, wherein the probe is comprised of a capture site consisting of a single nucleotide complementary to said target the ends of which are attached to two different double-stranded oligonucleotide regions wherein at least one of the oligonucleotide regions comprises detectable elements having a characteristic detection property and wherein the detectable elements are so arranged on the oligonucleotide region that the detection property is less detectable than when the same number of detectable elements are bound to a corresponding number of single nucleotides.

2. A method as claimed in claim 1 **characterised in that** it comprises the additional step of treating the used probe with a restriction enzyme and an exonuclease to liberate the detectable element(s) in a form in which can be detected.

3. A method as claimed in claim 2 **characterised in that** it further comprises the step of observing the detectable property exhibited by the liberated detectable element(s).

4. A method as claimed in any one of claims 1 to 3 **characterised in that** the detectable element(s) are fluorophore(s).

5. A method as claimed in any one of the preceding claims **characterised in that** the used probe comprises a restriction enzyme recognition site which has been formed by the attaching of the target to the capture site.

6. A method as claimed in any one of the preceding claims **characterised in that** the target is contacted with a mixture of four different biological probes each having a single-stranded nucleotide region comprising a different single nucleotide selected from (1) guanine, cytosine, adenine and thymine or (2) guanine, cytosine, adenine and uracil.

7. A method as claimed in claim 6 **characterised in that** each of the four probes has a different detectable element.

8. A method as claimed in claim 7 **characterised in that** the different detectable elements are fluorophores.

9. A method as claimed in any one of the preceding claims **characterised in that** the target comprises one of a stream of single nucleotides corresponding to the sequence of nucleotides in a DNA or RNA sample.

10. Use of a method according to any of the preceding claims to sequence DNA or RNA.

## Patentansprüche

1. Verfahren zum Nachweis eines Einzelnukleotid-Ziels, **dadurch gekennzeichnet, dass** es den Schritt des Anheftens des Ziels an eine Sonde unter Verwendung einer Polymerase und einer Ligase umfasst, um eine benutzte Sonde zu schaffen, die vollständig doppelsträngig ist, wobei die Sonde aus einer Einfangstelle besteht, die ein zu dem Ziel komplementäres Einzelnukleotid beinhaltet, dessen Enden an zwei verschiedene doppelsträngige Oligonukleotidregionen angeheftet sind, wobei mindestens eine der Oligonukleotidregionen detektierbare Elemente mit einer charakteristischen Detektionseigenschaft aufweist und wobei die detektierbaren Elemente so an der Oligonukleotidregion angeordnet sind, dass die Detektionseigenschaft weniger detektierbar ist als dann, wenn dieselbe Anzahl detektierbarer Elemente an eine entsprechende Anzahl von Einzelnukleotiden gebunden ist.

2. Verfahren, wie in Anspruch 1 beansprucht, **dadurch gekennzeichnet, dass** es den zusätzlichen Schritt des Behandelns der benutzten Sonde mit einem Restriktionsenzym und einer Exonuklease umfasst, um das/die detektierbare(n) Element(e) in einer Form freizusetzen, in welcher es/sie detektiert werden kann/können.

3. Verfahren, wie in Anspruch 2 beansprucht, **dadurch gekennzeichnet, dass** es außerdem den Schritt des Beobachtens der Detektionseigenschaft, wie durch das/die freigesetzte(n) detektierbare(n) Element(e) gezeigt, umfasst.

4. Verfahren, wie in einem der Ansprüche 1 bis 3 beansprucht, **dadurch gekennzeichnet, dass** das/die detektierbare(n) Element(e) Fluorophor(e) ist/sind.

5. Verfahren, wie in einem der vorangehenden Ansprüche beansprucht, **dadurch gekennzeichnet, dass** die benutzte Sonde eine Restriktionsenzym-Erkennungsstelle umfasst, welche durch das Anheften des Ziels an die Einfangstelle gebildet worden ist.

6. Verfahren, wie in einem der vorangehenden Ansprüche beansprucht, **dadurch gekennzeichnet, dass** das Ziel mit einer Mischung von vier verschiedenen biologischen Sonden kontaktiert wird, die jeweils eine einzelsträngige Nukleotidregion, umfassend ein unterschiedliches Einzelnukleotid, ausgewählt aus (1) Guanin, Cytosin, Adenin und Thymin oder (2) Guanin, Cytosin, Adenin und Uracil, aufweisen.

7. Verfahren, wie in Anspruch 6 beansprucht, **dadurch gekennzeichnet, dass** jede der vier Sonden ein unterschiedliches detektierbares Element ausweist.

8. Verfahren, wie in Anspruch 7 beansprucht, **dadurch gekennzeichnet, dass** die unterschiedlichen detektierbaren Elemente Fluorophore sind.

9. Verfahren, wie in einem der vorangehenden Ansprüche beansprucht, **dadurch gekennzeichnet, dass** das Ziel eines aus einem Strom von Einzelnukleotiden entsprechend der Sequenz von Nukleotiden in einer DNA- oder RNA-Probe umfasst.

10. Anwendung eines Verfahrens gemäß einem der vorangehenden Ansprüche zur Sequenzierung von DNA oder RNA.

## Revendications

1. Procédé de détection d'une cible formée d'un unique nucléotide, **caractérisé en ce qu'**il comprend l'étape consistant à fixer ladite cible à une sonde à l'aide d'une polymérase et d'une ligase afin de créer une sonde utilisée qui est totalement bicaténaire, dans lequel la sonde comprend un site de capture constitué d'un unique nucléotide complémentaire de ladite cible dont les extrémités sont fixées à deux régions oligonucléotidiques bicaténaires différentes, dans lequel au moins l'une des régions oligonucléotidiques comprend des éléments détectables possédant une propriété de détection caractéristique et dans lequel les éléments détectables sont agencés sur la région oligonucléotidique de telle sorte que la propriété de détection est moins détectable que lorsque le même nombre d'éléments détectables est lié à un nombre correspondant de nucléotides uniques.

2. Procédé selon la revendication 1, **caractérisé en ce qu'**il comprend l'étape supplémentaire de traitement de la sonde utilisée à l'aide d'une enzyme de restriction et d'une exonucléase afin de libérer le ou les éléments détectables sous une forme dans laquelle il(s) peu(ven)t être détecté(s).

3. Procédé selon la revendication 2, **caractérisé en ce qu'**il comprend, en outre, l'étape d'observation de la propriété détectable présentée par le ou les éléments détectables libérés.

4. Procédé selon l'une quelconque des revendications 1 à 3, **caractérisé en ce que** le ou les éléments détectables correspondent à un ou des fluorophores.

5. Procédé selon l'une quelconque des revendications précédentes, **caractérisé en ce que** la sonde utilisée comprend un site de reconnaissance d'une enzyme de restriction qui a été formé par la fixation de la cible au site de capture.

6. Procédé selon l'une quelconque des revendications précédentes, **caractérisé en ce que** la cible est mise en contact avec un mélange de quatre sondes biologiques différentes possédant chacune une région nucléotidique monocaténaire comprenant un nucléotide unique différent choisi parmi (1) la guanine, la cytosine, l'adénine et la thymine ou (2) la guanine, la cytosine, l'adénine et l'uracile.

7. Procédé selon la revendication 6, **caractérisé en ce que** chacune des quatre sondes possède un élément détectable différent.

8. Procédé selon la revendication 7, **caractérisé en ce que** les éléments détectables différents sont des fluorophores.

9. Procédé selon l'une quelconque des revendications précédentes, **caractérisé en ce que** la cible comprend un nucléotide d'un flux de nucléotides uniques correspondant à la séquence des nucléotides d'un échantillon d'ADN ou d'ARN.

10. Utilisation d'un procédé selon l'une quelconque des revendications précédentes pour le séquençage d'ADN ou d'ARN.
